# EUROPEAN PATENT APPLICATION

(11) **EP 4 159 192 A1**
(43) Date of publication of application: **05.04.2023**
(21) Application number: 21218282.8
(22) Date of filing: 30.12.2021
(51) Int. Cl.: A61K 9/00, A61K 31/137, A61P 25/00

(54) **DIMETHYLTRIPTAMINE-BASED NASAL SPRAY FOR THE PERSONALISED TREATMENT OF NEUROLOGICAL AND PSYCHIATRIC DISORDERS**

(30) Priority: 30.09.2021 UY 39445; 27.12.2021 US 202163293972 P
(71) Applicant: BIOMIND LABS INC, Toronto, Ontario M5J 2T9 (CA)
(72) Inventor: ANTALICH, Alejandro, Pando, Canelones (UY)
(74) Representative: Padial Martinez, Ana Belen

(57) **Abstract**

The present invention relates to a multidose nasal spray with mucoadhesive properties that gels in situ containing the psychedelic tryptamines DMT or 5-MeO-DMT or pharmaceutically acceptable salts or derivatives thereof, which can be used for personalized treatment of neurological and psychiatric disorders.

## Description

### FIELD OF THE INVENTION

The present invention discloses a multidose nasal spray containing N, N-dimethyltryptamine (DMT) or 5-methoxy-N, N-dimethyltryptamine (5-MeO-DMT) or pharmaceutically acceptable salts or derivatives thereof for the personalized treatment of neurological and psychiatric disorders. Also disclosed are methods of preparing the nasal spray.

### SUMMARY

One general aspect of the invention discloses a nasal spray including i) N,N-dimethyltryptamine or 5-methoxy-N, N-dimethyltryptamine, or a pharmaceutically acceptable salt or derivative thereof, ii) a gelling agent, and iii) a mucoadhesive agent. In this embodiment, the nasal spray has a viscosity suitable for spraying, and the nasal spray gels upon application and adheres to the nasal mucosa.

In an embodiment of the invention, the gelling agent can include poloxamer, gellan gum, xanthan gum, sodium hyaluronate, iota-carrageenan, carbomer, agar agar, polyvinylpyrrolidone, polyethylene oxide, or combinations thereof.

In an embodiment of the invention, the gelling agent includes poloxamer, and the nasal spray can include 5%-30% w/v of the poloxamer. In this embodiment, the poloxamer can be selected from poloxamer 407, poloxamer 188, and combinations thereof. In an exemplary embodiment, the nasal spray can include 20% w/v poloxamer 407 and 10% w/v poloxamer 188.

In an embodiment of the invention, the mucoadhesive agent includes hydroxypropyl methylcellulose (HPMC), chitosan, polycaprolactone, polycarbophil-cysteine, or combinations thereof. In this embodiment, the nasal spray can include 0.1-1% w/v of the mucoadhesive agent. In an exemplary embodiment, the nasal spray includes 0.55% w/v HPMC K-100.

In an embodiment of the invention, the nasal spray can have a final viscosity on the nasal mucosa of between 50cP and 300cP. In an exemplary embodiment, the final viscosity on the nasal mucosa of can be between 50cP and 200cP.

In an embodiment of the invention, the nasal spray gels at a temperature of between 30°C to 34°C.

In an embodiment of the invention, the nasal spray can include an inhibitor of monoamine oxidase inhibitor (MAOI), such as beta-carbolines, resveratrol, curcumin, flavonoids, quercetin, crysine, shawls, and analogs or derivatives thereof.

Another general aspect of the invention discloses a method of treating a neurological and/or psychiatric disorder by administering to an individual in need thereof the nasal spray.

Another general aspect of the invention discloses a multidose nasal spray container that includes the nasal spray.

Another general aspect of the invention discloses a method of making the nasal spray of claim 1, including the steps of:
i) dissolving N,N-dimethyltryptamine, 5-methoxy-N, N-dimethyltryptamine, or a pharmaceutically acceptable salt or derivative thereof, in a physiological serum to prepare a solution;
ii) cooling the solution in an ice bath;
iii) stirring the solution while adding the mucoadhesive agent to obtain a first intermediate;
iv) adding the gelling agent to the first intermediate to obtain a second intermediate and stirring for 2 hours; and
v) cooling the second intermediate to obtain the nasal spray.

In this embodiment, the physiological serum can include glycerin and benzalkonium chloride. In this embodiment, the gelling agent can include a poloxamer, and the mucoadhesive agent can include hydroxypropyl methylcellulose (HPMC).

In this embodiment, step iv) of adding the gelling agent can include the steps of (a) adding a first gelling agent with stirring until dissolved followed by (b) adding a second gelling agent with stirring until dissolved.

### BACKGROUND OF THE INVENTION

Both N, N-dimethyltryptamine (DMT) and 5-methoxy-N, N-dimethyltryptamine (5-MeO-DMT) belong to the psychoactive indole alkylamine family and are naturally biosynthesized by various natural organisms.¹

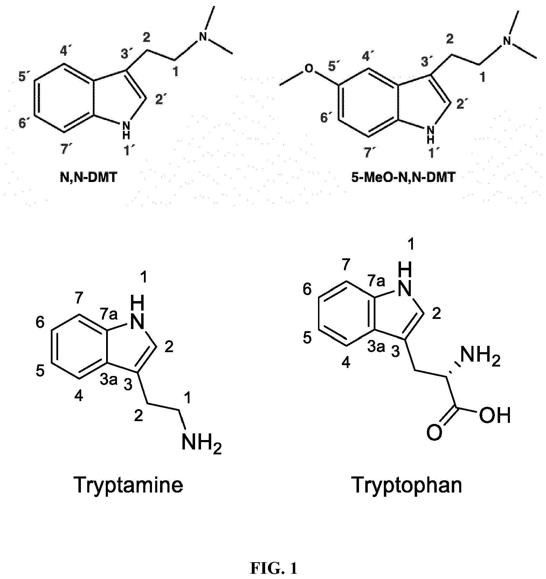

As can be seen from their atomic structures (**Figure 1**), both DMT and 5-MeO-DMT are molecular analogs of tryptamine (3-(2-Aminoethyl) indole, 2-(3-Indole) ethylamine), which itself is a molecular analog of tryptophan. Tryptophan is an essential amino acid that comes from the diet in animals and is produced endogenously in plants.

Scientific studies have shown that in humans both DMT and 5-MeO-DMT are endogenously synthesized by the retina and pineal gland, and that they have also been detected in blood, urine, and cerebrospinal fluid.^{2,3,4,5,6}

There is evidence that several cultures used plants containing DMT and 5-MeO-DMT for medicinal, psychological and entheogenic purposes for at least 4 millennia.^{7,8}

Both DMT and 5-MeO-DMT are currently undergoing an increasing number of scientific studies and clinical trials for the treatment of a variety of psychiatric disorders, such as depression, anxiety, headache, and obsessive-compulsive disorder.^{9,10,11,12,13}

### BRIEF DESCRIPTION OF THE DRAWINGS

Figure 1 shows the chemical structures of DMT and its analogue 5-methoxy-N, N-dimethyltryptamine (5-MeO-DMT), as well as tryptamine and tryptophan.
Figure 2 shows a graphic scheme of a nasal spray.
Figure 3 shows a graphic scheme of the mucoadhesive properties of the nasal spray.

### DETAILED DESCRIPTION OF THE INVENTION

As used herein, the term "pharmaceutically acceptable salts or derivatives thereof" herein refers to those salts or derivatives which possess the biological effectiveness and properties of the salified or derivatized compound and which do not produce adverse reactions when administered to a mammal, preferably a human. The pharmaceutically acceptable salts may be inorganic or organic salts; examples of pharmaceutically acceptable salts include but are not limited to: carbonate, hydrochloride, hydrobromide, sulphate, hydrogen sulphate, citrate, maleate, fumarate, tifluoroacetate, 2-naphthalenesulphonate, and para-toluenesulphonate. Further information on pharmaceutically acceptable salts can be found in Handbook of pharmaceutical salts, P. Stahl, C. Wermuth, WILEY-VCH, 127-133, 2008. Pharmaceutically acceptable derivatives include esters, the ethers, and N-oxides.

DMT and 5-MeO-DMT act as non-selective serotonin agonists at the 5-HT2A, 5-HT2C, 5-HT1A receptors, among others. The 5-HT system is associated with cognition, memory, emotions, circadian rhythm, alertness, and pain inhibition.^{14,15,16,17}

It has been shown that both molecules suffer a rapid first-pass metabolism effect due to deamination mediated by the enzyme monoamine oxidase A (MAO-A), which renders these molecules inactive when orally administered. This inactivation poses a major problem that must be solved to facilitate administration of DMT and 5-MeO-DMT to patients.^{18,19,20}

An individual's rate of metabolization of these tryptamines impacts the pharmacodynamics; that is, the physiological, biochemical, and molecular effects of the DMT and/or 5-MeO-DMT. Naturally, in case of psychedelic substances this impacts the quality of the acute psychedelic experience and, therefore, influences the therapeutic result. People metabolize drugs at different speeds, which is due to many factors, such as the speed at which the drug is transported to cells, the rate at which the drug decomposes within cells, and the speed at which the drug is excreted. Genetic characteristics also influence these processes since they are mediated by enzymes and transport proteins. Environmental factors also play an important role. Diet, for example, can increase the production of a relevant enzyme or provide cofactors that modulate the activity of different enzymes, particularly when administered orally.²¹ From the pharmacokinetic standpoint, these molecules have a rapid onset of action and relatively short duration.

Recreationally, DMT and/or 5-MeO-DMT are exposed to high temperatures for a prolonged period of time and inhaled. This process induces the formation of degradation products, which are also inhaled. These degradation products have unknown pharmacological effects and are potentially harmful. This highlights the need to design and develop pharmaceutical formulations of DMT and 5-MeO-DMT that employ strategies to avoid exposure to by-products from degradation, improve bioavailability and, therefore, ensure therapeutic action.

Oral administration of drugs, intended for systemic (peroral) absorption, is preferred by patients for comfort and safety reasons. However, oral administration has several disadvantages, including exposing the drug to an aggressive gastrointestinal environment, which can degrade bioactive molecules and limits their absorption and reduces pharmacotherapy. Some of these disadvantages can be partially overcome through parenteral administration, which is typically avoided by patients due to its invasiveness.²²

Intranasal transmucosal administration (use of the mucosa that covers the nostrils) of drugs is an attractive alternative to the oral and parenteral pathways and comparatively has many advantages. Advantages of intranasal administration include, for example, the reduction of first-pass metabolism, prevention of gastrointestinal degradation, and direct systemic administration to the central nervous system resulting in rapid onset of action of the drug.^{23,24}

In addition, intranasal transmucosal administration allows for a rapid onset of action, ease of administration and a decrease in adverse effects, since it is possible to decrease drug doses compared to those necessary to achieve the same action by other forms of systemic administration, such as oral administration.

WO 2020/245133A1 and WO 2021/089872A1 disclose compositions of DMT and deuterated analogs thereof as active ingredients to treat psychiatric or psychocognitive disorders. WO 2021/030571A1 describes methods to prevent or treat psychological disorders by administering serotonin receptor agonists which are administered separately, sequentially, or simultaneously in combination with a 2A receptor antagonist. WO 2019/081764A1 provides a combined product for the treatment and / or prevention of psychiatric and / or neurological disorders. This combined product contains within its formulation a compound with psychedelic effects, such as DMT. However, the compositions and methods described in these patent applications do not teach a defined and therapeutically effective dosage regimen.

Intranasal administration of drugs effectively achieves therapeutic concentrations directly in the brain through the olfactory and trigeminal nerves without going through the blood-brain barrier and reduces the hepatic first step effect, thereby ensuring a faster onset of pharmacological activity.²⁵

Therefore, intranasal administration has great potential in the development of new pharmaceutical products. This invention presents the development of a pharmaceutical form of DMT or 5-MeO-DMT or pharmaceutically acceptable salts or derivatives thereof, which are suitable for intranasal administration, as well as personalized treatment of neurological and psychiatric disorders. Through this pharmaceutical form, a standardized and reproducible target dose is provided.

US 7,090,830, EP 1389098, and US 8,955,512 disclose thermally generated condensation aerosols; however, none employ DMT or 5-MeO-DMT. WO 2002/094216 teaches administration of stimulants, specifically ephedrine or fenfluramine, through the inhalation route.

WO 2021/170614A1 teaches an aerosol containing 5-MeO-DMT; however, certain limitations of the nasal pathway are not considered, such as rapid elimination of the formulation through mucociliary clearance, which results in limited retention time. To overcome these limitations, mucoadhesive polymers can be used to increase contact time and improve drug permeation.^{26,27,28}

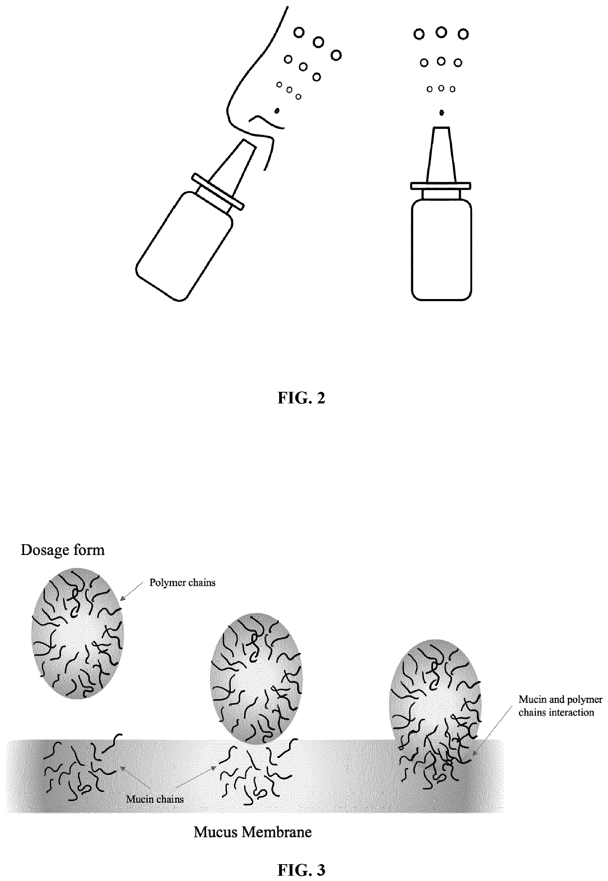

While the use of a defined-dose spray is advantageous, other factors must be considered during formulation. For example, the formulation should have a reduced viscosity, but this can result in problems with drug retention on the mucosa after administration. Accordingly, the present invention provides a nasal spray (Figure 2) with *in situ* gelling using polymers that gel at body temperature, thereby providing a therapeutically effective dose to a patient that is well retained on the nasal mucosa.

The therapeutic effects of the dimethyltryptamines DMT, 5-MeO-DMT, and salts thereof depend on the quality of the acute effects of the psychedelic experience felt. However, the phenomenology of these effects depends to a large extent on the effective dosage, which is partly related to the physiological mechanisms of the particular disease, as well as the individual's metabolization of the substance. Therefore, to achieve the desired efficacy of dimethyltryptamines, there is a need to evaluate the effective concentration of dimethyltryptamine together with treatment outcomes to ensure that the most effective dose is being administered. This invention discloses a multidose nasal spray for the personalized treatment of neurological and psychiatric disorders. The nasal spray contains at least one of the psychedelic tryptamines DMT or 5-MeO-DMT or pharmaceutically acceptable salts or derivatives thereof. The nasal spray includes one or more gelling agents in combination with one or more mucoadhesive agents to provide mucoadhesive properties upon administration. In exemplary embodiments, the gelling agent or agents can be present in an amount of 5-50% w/v, for example 10-40% w/v, or about 30% w/v. In exemplary embodiments, the mucoadhesive agent is present in an amount of 0.1-1% w/v, for example, about 0.2%, about 0.3%, about 0.4%, about 0.5%, about 0.6%, about 0.7%, about 0.8%, about 0.9%, or about 1.0%. In an exemplary embodiment, the mucoadhesive agent is present at about 0.55% w/v. The one or more gelling agents can be a polymer and the one or more mucoadhesive agents can be hydroxypropyl methylcellulose (HPMC), (**Figure 3**) which are both dispersed in the bulk phase of the product that behaves like a liquid in order to facilitate administration, but forms a gel at body temperature that adheres to the nasal mucosa of the nasal cavity to improve the performance of the pharmaceutical formulation.

In exemplary embodiments, the gelling agent includes one or more poloxamers alone or in combination with other natural and synthetic gelling agents. Other natural or synthetic gelling agents that can be used in place of or together with poloxamers include, for example, carrageenan, gums, hyaluronic acid, carbomers, derivatives of polyacrylic acid and other gelling polymers. Exemplary gelling agents include agar agar, polyvinylpyrrolidone, polyethylene oxide, gellan gum (up to about 0.2%), xanthan gum (up to about 0.2%), sodium hyaluronate (up to about 0.1%), iota-carrageenan (up to about 0.2%), and carbomers (up to about 0.5%).

In exemplary embodiments, the mucoadhesive agent includes HPMC, such as HMPC K-100. Other natural or synthetic mucoadhesive agents can include, for example, chitosan, polycaprolactone, or polycarbophil-cysteine.

The formulation selectively gels at a temperature of 30°C to 34°C. The gelling agent is added in an amount to achieve a nasal spray with a viscosity on the nasal mucosa between 50cP and 300cP, for example between 75cP and 275cP.

In exemplary embodiments, the combination of the one or more gelling agents with the one or more mucoadhesive provides a gellable formulation, having with a viscosity suitable for spraying and provides mucosal adhesion in the nasal cavity by gelling at a temperature of 30°C to 34°C. In exemplary embodiments, after gelling, the materials achieve a viscosity on the nasal mucosa between 50cP and 300cP, preferably between 50cP and 200cP. Exemplary embodiments can include about 10-40% w/v of one or more poloxamers, for example, Poloxamer 407 and/or Poloxamer 188, and 0.55% w/v HPMC.

Poloxamer 407 can be present in an amount of from about 10% (w/v) to about 40%, about 15% to about 25 % or about 20%. Poloxamer 188 can be present in an amount of about 5% (w/v) to about 40%, about 5% to about 15 % or about 10%. An exemplary embodiment includes about 10% w/v Poloxamer 407 and/or 20% w/v Poloxamer 188. HMPC can be present in an amount of from about 0.1% (w/v) to about 1%, about 0.2%, about 0.3%, about 0.4%, about 0.5%, about 0.6%, about 0.7%, about 0.8%, about 0.9%, or about 1.0%. An exemplary embodiment includes about 0.55% w/v HPMC.

The nasal spray composition can be formulated in an isotonic medium with NaCl and/or other physiologically acceptable salts. N, N-dimethyltryptamine and/or 5-methoxy-N, N-dimethyltryptamine are preferably in the form of free bases and/or pharmaceutically acceptable salts or derivatives thereof.

The formulation can be administered in a multidose nasal spray container of multiple unit doses (multiple single dose).

The formulation can further include an inhibitor of monoamine oxidase (i.e MAOI), such as natural or synthetic beta-carbolines, resveratrol, curcumin, flavonoids, such as quercetin and chrysin, among others. The MAOI can be obtained from natural sources or by chemical synthesis. Synthetic MAOI derivatives include, but are not limited to, substituted shawls, or analogs of curcumin and analogs of resveratrol.

In general, nasal sprays of the present invention are prepared by a method that includes the steps of:
i) Adding N,N-dimethyltryptamine, 5-methoxy-N, N-dimethyltryptamine, or a pharmaceutically acceptable salt or derivative thereof, to a physiological serum to obtain a solution of active. The physiological serum may include additional additives, such as glycerin and benzalkonium chloride;
ii) Other additives, for example a MAOI, can be added and the solution is then cooled in an ice bath;
iii) While stirring, one or more mucoadhesive agents is added to the solution to obtain a first intermediate;
iv) The one or more gelling agents are then added to the first intermediate to obtain a second intermediate, which is stirred for a predetermined amount of time, which is typically 2 hours. The one or more gelling agents may be combined and added or, more typically, added sequentially. For example, Poloxamer 188 is slowly added until complete dissolution is obtained, and a second gelling agent, for example Poloxamer 407, is then slowly added until complete dissolution is obtained;
v) The second intermediate is then cooled to obtain the nasal spray. Preferably, the second intermediate is cooled and stored at a reduced temperature, for example, 8°C, to obtain the nasal spray.

The invention is illustrated in more detail below in the following non-limiting examples.

### EXAMPLES

### EXAMPLES 1-3- FORMULATIONS

Three exemplary non-limiting formulation examples are provided in Table 1 below, along with their DMT concentrations, gelling temperatures (T Gel), and viscosities.

| Table 1 | | | | | | |
|---|---|---|---|---|---|---|
| Example | Concentration (mg/mL) | P407 (w/v) | P188 (w/v) | HPMC (w/v) | T Gel (°C) | Viscosity (cP) |
| 1 | 6.6 | 20% | 5% | 0.55% | 27.5 | 165.7 |
| 2 | 10 | 15% | 5% | 0.55% | 38.4 | 81.1 |
| 3 | 20 | 18% | 10% | 0.55% | 32.8 | 254.4 |

### EXAMPLES 4-5 - METHODS OF PREPARING

### EXAMPLE 4

First, glycerin (final concentration of 1% w/v) and benzalkonium chloride (final concentration of 0.02% w/v) are dissolved in 25 mL of physiological serum. Next, 400 mg of DMT fumarate is added to the serum. Then, with the serum stirring vigorously in an ice bath, HPMC K100 (final concentration of 0.55% w/v) is slowly added to the to the serum.

Next, Poloxamer 188 (final concentration of 10% w/v) is slowly added to the serum. Upon complete dissolution of Poloxamer 188, Poloxamer 407 (final concentration of 20% w/v) is slowly added to the serum. Vigorous stirring is continued for an additional 2 hours, and the final solution is stored at 8°C overnight.

### EXAMPLE 5

First, glycerin (final concentration of 1% w/v) and benzalkonium chloride (final concentration of 0.02% w/v) are dissolved in 25 mL of physiological serum. Next, 500 mg of micronized DMT is added to the serum. Then, with the serum stirring vigorously in an ice bath, HPMC K100 (final concentration of 0.55% w/v) is slowly added to the to the serum.

Next, Poloxamer 188 (final concentration of 10% w/v) is slowly added to the serum. Upon complete dissolution of Poloxamer 188, Poloxamer 407 (final concentration of 20% w/v) is slowly added to the serum. Vigorous stirring is continued for an additional 2 hours, and the final solution is stored at 8°C overnight.

### REFERENCES

1 Carbonaro T.M. and Gatch M.B., Brain Research Bulletin, 2016; 126:74-88: "Neuropharmacology of N, N-dimethyltryptamine".
2 Narasimhachari N. et al., Biological Psychiatry, 1971; 3:21-23: "N,N-dimethylated indoleamines in blood".
3 Oon M.C. et al., Psychopharmacology, 1977; 54:171-175: "Factors affecting the urinary excretion of endogenously formed dimethyltryptamine in normal human subjects".
4 Smythies J.R. et al, Biological Psychiatry, 1979; 14:549-556: "Identification of dimethyltryptamine and O-methylbufotenin in human cerebrospinal fluid by combined gas chromatography/mass spectrometry".
5 Sitaram B.R. et al., Analytical biochemistry, 1983; 128:11-20: "The ion-pair extraction, purification, and liquid chromatographic analysis of indolealkylamines in human urine".
6 Forsstrom T., Tuominen J. and Karkkainen J., Scandinavian journal of clinical and laboratory investigation, 2001; 61:547-556: "Determination of potentially hallucinogenic N-dimethylated indoleamines in human urine by HPLC/ESI-MS-MS".
7 Metzner R., New York: Thunder's Mouth Press, 1999: "Ayahuasca: hallucinogens, consciousness, and the spirit of nature".
8 McKenna D.J. et al., Journal of Ethnopharmacology, 1984; 10 (2): 195-223: "Monoamine oxidase inhibitors in South American hallucinogenic plants: tryptamine and beta-carboline constituents of ayahuasca".
9 Strassman R.J., Journal of Psychoactive Drugs, 1991; 23:29-38: "Human hallucinogenic drug research in the United States: a present-day case history and review of the process".
10 Strassman R.J., The Journal of nervous and mental disease, 1995; 183:127-38: "Hallucinogenic drugs in psychiatric research and treatment. Perspectives and prospects".
11 Davis A.K. et al., The American Journal of Drug and Alcohol Abuse, 2019; 45:161-169: "5-Methoxy-N,N-Dimethyltryptamine (5-MeO-DMT) Used in a Naturalistic Group Setting Is Associated with Unintended Improvements in Depression and Anxiety".
12 Osório, F. de L. et al., Revista Brasileira de Psiquiatria, 2015, 37(1): 13-20: "Antidepressant Effects of a Single Dose of Ayahuasca in Patients with Recurrent Depression: A Preliminary Report".
13 Dos Santos R.G. and Hallak, J.E.C., Neuroscience and biobehavioral reviews, 2020, 108:423-434: "Therapeutic use of serotoninergic hallucinogens: A review of the evidence and of the biological and psychological mechanisms".
14 Szara S., Experientia, 1956, 12:441-442: "Dimethyltryptamin: its metabolism in man; the relation to its psychotic effect to the serotonin metabolism".
15 Franzen F, and Gross H., Nature, 1965; 206:1052: "Tryptamine, N,N-dimethyltryptamine, N,N-dimethyl-5-hydroxytryptamine and 5-methoxytryptamine in human blood and urine".
16 Arturo A. et al., Journal of nuclear medicine, 2011; 52:970-977: "In Vivo Long-Term Kinetics of Radiolabeled N,N-Dimethyltryptamine and Tryptamine".
17 McKenna, D.J. et al., Neuropharmacology, 1990; 29:193-198: "Differential interactions of indolealkylamines with 5-hydroxytryptamine receptor subtypes".
18 Luethi, D. and Liechti, M.E., The international journal of neuropsychopharmacology, 2018; 21:926-931: "Monoamine transporter and receptor interaction profiles in vitro predict reported human doses of novel psychoactive stimulants and psychedelics".
19 Szabo, A. et al., PLoS One, 2014; 9:e106533: "Psychedelic N,N-dimethyltryptamine and 5-methoxy-N,N-dimethyltryptamine modulate innate and adaptive inflammatory responses through the sigma-1 receptor of human monocyte-derived dendritic cells".
20 Sitaram, B.R. et al.; Biochemical Pharmacology, 1987; 36:1509-1512: "In vivo metabolism of 5-methoxy-N, N-dimethyltryptamine and N,N-dimethyltryptamine in the rat".
21 Callaway, J.C., Journal of Psychoactive Drugs, 2005, 37(2):157-61, "Fast and Slow Metabolisers of Hoasca".
22 Dubey, S.K. et al., Drug Discovery Today, 2021; 26(4):931-950: "Oral peptide delivery: challenges and the way ahead".
23 Yunjie Xia, et al., Drug Development and Industrial Pharmacy, 2020: 46(5):697-705: "Performance and toxicity of different absorption enhancers used in the preparation of poloxamer thermosensitive in situ gels for ketamine nasal administration".
24 Majcher M.J. et al., Journal of Controlled Release, 2021; 330:738-752: "In situ-gelling starch nanoparticle (SNP)/O-carboxymethyl chitosan (CMCh) nanoparticle network hydrogels for the intranasal delivery of an antipsychotic peptide".
25 Johnson N. et al., Molecular pharmaceutics, 2010; 7(3):884-893: "Trigeminal pathways deliver a low molecular weight drug from the nose to the brain and orofacial structures".
26 Swamya N.G.N. and Abbas Z., Asian Journal of Pharmaceutical Sciences, 2012, 7(3):168-180: "Mucoadhesive in situ gels as nasal drug delivery systems: an overview".
27 Shaikh R. et al., Journal of Pharmacy & Bioallied Sciences, 2011; 1:89-100: "Mucoadhesive drug delivery systems".
28 Russo, E. and Villa, C., Pharmaceutics, 2019; 11:671: "Poloxamer Hydrogels for Biomedical Applications".

## Claims

1. A nasal spray comprising i) N,N-dimethyltryptamine or 5-methoxy-N, N-dimethyltryptamine, or a pharmaceutically acceptable salt or derivative thereof, ii) a gelling agent, and iii) a mucoadhesive agent, wherein the nasal spray has a viscosity suitable for spraying, and wherein the nasal spray gels upon application and adheres to the nasal mucosa.

2. The nasal spray of claim 1, wherein the gelling agent comprises poloxamer, gellan gum, xanthan gum, sodium hyaluronate, iota-carrageenan, carbomer, agar agar, polyvinylpyrrolidone, polyethylene oxide, or combinations thereof.

3. The nasal spray of claim 1 or 2, wherein the gelling agent comprises poloxamer.

4. The nasal spray of claim 3, wherein the nasal spray comprises 5%-30% w/v of the poloxamer.

5. The nasal spray of claim 3 or 4, wherein the poloxamer is selected from the group consisting of poloxamer 407, poloxamer 188, and combinations thereof.

6. The nasal spray of claim 5, wherein the nasal spray comprises 20% w/v poloxamer 407 and 10% w/v poloxamer 188.

7. The nasal spray of claim 1, wherein the mucoadhesive agent comprises hydroxypropyl methylcellulose (HPMC), chitosan, polycaprolactone, polycarbophil-cysteine, or combinations thereof.

8. The nasal spray of claim 1 or 7, wherein the nasal spray comprises 0.1-1% w/v of the mucoadhesive agent.

9. The nasal spray of claim 8, wherein the nasal spray comprises 0.55% w/v HPMC K-100.

10. The nasal spray of claim 1, wherein the nasal spray has a final viscosity on the nasal mucosa of between 50cP and 300cP.

11. The nasal spray of claim 10, wherein the nasal spray has a final viscosity on the nasal mucosa of between 50cP and 200cP.

12. The nasal spray of claim 1, wherein the nasal spray gels at a temperature of between 30°C to 34°C.

13. The nasal spray of claim 1, further comprising an inhibitor of monoamine oxidase inhibitor (MAOI).

14. The nasal spray of claim 13, wherein the MAOI is selected from the group consisting of beta-carbolines, resveratrol, curcumin, flavonoids, quercetin, crysine, shawls, and analogs or derivatives thereof.

15. A method of treating a neurological and/or psychiatric disorder comprising administering to an individual in need thereof the nasal spray of claim 1.

16. A multidose nasal spray container comprising the nasal spray of claim 1.

17. A method of making the nasal spray of claim 1, comprising the steps of:
i) dissolving N,N-dimethyltryptamine, 5-methoxy-N, N-dimethyltryptamine, or a pharmaceutically acceptable salt or derivative thereof, in a physiological serum to prepare a solution;
ii) cooling the solution in an ice bath;
iii) stirring the solution while adding the mucoadhesive agent to obtain a first intermediate;
iv) adding the gelling agent to the first intermediate to obtain a second intermediate and stirring for 2 hours; and
v) cooling the second intermediate to obtain the nasal spray.

18. The method of claim 17, wherein the physiological serum comprises glycerin and benzalkonium chloride.

19. The method of claim 17, wherein the gelling agent comprises a poloxamer.

20. The method of claim 17, wherein the mucoadhesive agent comprises hydroxypropyl methylcellulose (HPMC).

21. The method of claim 17, wherein step iv) of adding the gelling agent comprises the steps of (a) adding a first gelling agent with stirring until dissolved followed by (b) adding a second gelling agent with stirring until dissolved.
